# EUROPEAN PATENT APPLICATION

(11) **EP 1 484 340 A1**
(43) Date of publication of application: **08.12.2004**
(21) Application number: 04076687.5
(22) Date of filing: 01.08.1991
(51) Int. Cl.: C07K 16/18

(54) **Antibodies to a novel mammalian protein associated with uncontrolled cell division**

(30) Priority: 01.08.1990 US 561989
(62) Divisional of application: 91914930.2
(71) Applicant: THE JOHNS HOPKINS UNIVERSITY, Baltimore, MD 21205-2109 (US)
(72) Inventor: Pasternack, Gary R., Baltimore, Maryland 21210 (US)
(74) Representative: Jones, Nicholas Andrew

(57) **Abstract**

A preparation of antibodies which specifically binds to a mammalian protein comprising an amino acid sequence as shown in Figure 9. Also provided is a diagnostic method for predicting malignant potential of lymphoid and epithelial tumors, comprising obtaining a sample from human lymphoid or epithelial tissue and contacting the sample with antibodies which specifically bind a mammalian protein found in the nucleus of A₂₀ cells having a molecular weight of about 35 kDa as determined by SDS-PAGE, wherein said mammalian protein is a substrate for casein kinase II *in vitro.*

## Description

### BACKGROUND OF THE INVENTION

A number of proliferation-associated nuclear proteins have been described (Wheely and Baserga, 1977, Cell Biol. Int. Rep., vol. 1, p. 13-21; Tan et al., 1987, Nucleic Acids Res., vol. 15, pp. 9299-9308; Gomez-Marquez et al., 1989, J. Biol. Chem., vol. 264, pp. 8451-8454; Feuerstein et al.. 1988, J. Cell Biol., vol. 107, pp. 1629-1642: Shawyer et a., 1989, J. Biol. Chem., vol. 264, pp. 1046-1050; Jaskulski et al., 1988, J. Biol. Chem., vol. 263, pp. 10175-10179). Some, such as proliferating cell nuclear antigen (Tan et al., 1987, Nucleic Acids Res.. vol. 15, pp. 9299-9308; Jaskulski et al., 1988, J. Biol. Chem., vol. 263, pp. 10175-10179), a co-factor of DNA polymerase delta, participate directly in proliferation. Still others, such as KI-67, are of unknown function (Gerdes et al., 1984, J. Immunol., vol. 133, pp. 1710-1715). In cases involving nuclear phosphoproteins, phosphorylation and dephosphorylation through systems of kinases and phosphatases may be important in coordinating molecular functions (Morla et al., 1989. Cell, vol. 58, pp. 193-203; Chen et al., 1989, Cell, vol. 58. pp. 1193-1198: Cooper and Whyte, 1989, Cell, vol. 58, pp. 1009-1011). While these proteins are expressed under normal physiological conditions, there is the possibility that derangement of the expression of one or more of these proteins could be involved in the growth and development of malignancies.

There is a continuing need in the medical arts for new means to diagnose and prognose cancers. Quick and simple methods can lead to more widespread cancer testing and earlier diagnoses, which can save lives by allowing therapy at earlier stages of the disease process.

### SUMMARY OF THE INVENTION

It is an object of the invention to provide substantially purified preparations of mammalian proteins which are diagnostic and prognostic of human cancers.

It is another object of the invention to provide preparations of antibodies which are immunoreactive with mammalian proteins and which are useful in the diagnosis and prognosis of human cancers.

It is yet another object of the invention to provide methods of producing and purifying mammalian proteins which are diagnostic and prognostic of human cancers.

It is still another object of the invention to provide nucleic acid probes which are complementary to mRNA encoding proteins which are diagnostic and prognostic of human cancers.

It is an object of the invention to provide diagnostic methods for predicting malignant potential of lymphoid and epithelial tumors.

These and other objects of the invention are provided by one or more of the embodiments which are described below. In one embodiment a substantially purified preparation of mammalian protein is provided which: has a molecular weight of about 35 kD; binds to myosin filaments; and is a substrate for casein kinase II in vitro.

In another embodiment of the invention substantially purified preparations of a mammalian protein are provided which protein has a molecular weight of about 32 kD and is immunoreactive with antibodies directed against pp35; and is a substrate for casein kinase II in vitro.

In still another embodiment of the invention substantially purified preparations of a mammalian protein are provided which protein has a molecular weight of about 42 kD and is immunoreactive with antibodies directed against pp35.

In another embodiment of the invention a preparation of antibodies is provided which is immunoreactive with a native mammalian protein which: has a molecular weight of about 35 kD; binds to myosin filaments; and is a substrate for casein kinase II in vitro.

In still another embodiment of the invention a preparation of antibodies is provided which is immunoreactive with a mammalian protein which: has a molecular weight of about 32 kD: is immunoreactive with antibodies directed against pp35; and is a substrate for casein kinase II in vitro.

In still another embodiment of the invention a preparation of antibodies is provided which is immunoreactive with a mammalian protein which: has a molecular weight of about 42 kD; and is immunoreactive with antibodies directed against pp35.

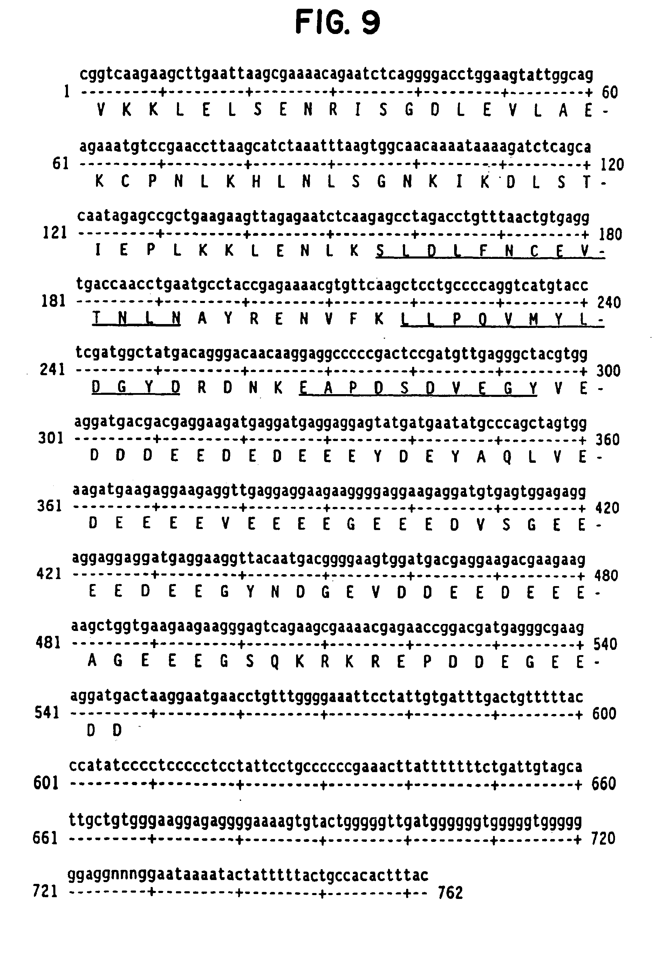

In yet another embodiment a preparation of antibodies is provided which is immunoreactive with a polypeptide comprising amino acid sequences as shown in Figure 9.

In still another embodiment of the invention a preparation of antibodies is provided which is produced by immunizing animals with an imunogen comprising a polypeptide comprising amino acid sequences as shown in Figure 9.

In another embodiment a method is provided of purifying mammalian proteins pp35 and pp32 comprising: lysing cells in a detergent and low-ionic strength buffer to form a cell lysate; separating components of the cell lysate by DEAE-cellulose chromatography and selecting desired components; separating the desired components into fractions by HPLC anion-exchange chromatography and selecting desired fractions; separating the desired fractions into constituents by HPLC hydroxylapatite chromatography and selecting desired constituents, desired constituents having a molecular weight of about 35 or about 32 kD.

In still another embodiment of the invention, nucleic acid primers are provided for amplifying sequences encoding pp32, pp35 or pp42.

In another embodiment of the invention a nucleic acid probe is provided which is complementary to mRNA encoding pp32, pp35 or pp42.

In still other embodiment of the invention diagnostic methods are provided for predicting malignant potential of lymphoid and epithelial tissues comprising: providing a section of human lymphoid or epithelial tissue; determining levels of or intracellular sites of expression of a gene product expressed from a gene selected from the group consisting of: pp32, pp35, and pp42.

In yet another embodiment of the invention a method of producing a preparation of a mammalian protein selected from the group consisting of pp32, pp35, and pp42 is provided comprising: culturing a mammalian lymphoblastoid cell line; collecting the mammalian protein from the nucleus of cells of the cell line. These and other embodiments of the invention will be described in more detail below. The present invention thus provides the arts of oncology and pathology with entirely new diagnostic tools for determining the malignant potential of lymphoid and epithelial tumors.

### BRIEF DESCRIPTION OF THE DRAWINGS

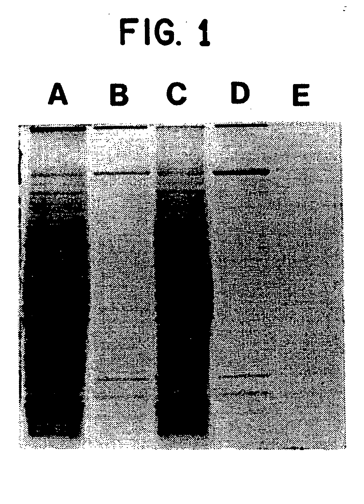

Figure 1 shows myosin-affinity separation of pp35. A lysate prepared from A₂₀ cells (8 mg total protein) was incubated with 1 mg of cross-linked myosin for 30 min at 4°. The myosin was pelleted at 16,000 x g for 10 min., the supernatant removed, and the pellet washed three times in lysis buffer. The pellet was then resuspended in lysis buffer containing 1 M KCl and incubated at 4° for 15 min. After incubation, the myosin was again pelleted. The figure represents total A₂₀ lysate, lane A; myosin precipitate, lane B; myosin supernatant, lane C; KCl myosin precipitate, lane D; and KCl myosin extract, lane E. The figure shows a Coomassie-stained 7-15% gel, Myosin pellets were prepared for electrophoresis using Laemmli solubilizing buffer without β-mercaptoethanol to avoid solubilizing the disulfide cross-linked myosin; 8-mercaptoethanol was added after re-centrifugation and prior to electrophoresis.

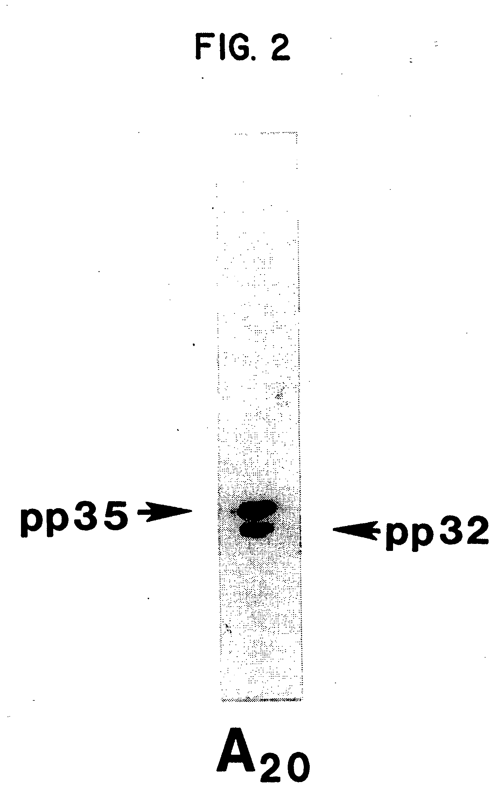

Figure 2 shows detection of pp35 and pp32 by antibody to denatured pp35 (pp35d). The figure represents a Western blot of A₂₀ cell lysate developed using anti-pp35d and ¹²⁵I-protein A.

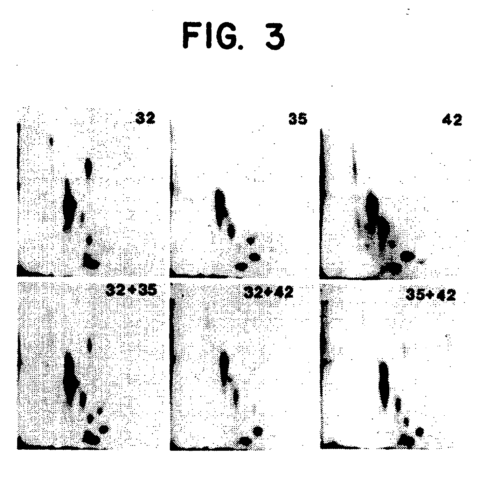

Figure 3 shows analysis of pp42, pp35, and pp32 by peptide mapping. pp42, pp35, and pp32 were each excised from Coomassie-stained gels, iodinated, digested with α-chymotrypsin, applied to cellulose sheets, and subjected to high-voltage electrophoresis along the horizontal dimension, and to thin-layer chromotography in the vertical dimension. The figure represents the resulting autoradiographs. The top row shows the map of each protein individually, while the bottom row illustrates a mixing experiment in which equal amounts of radioactivity of the indicated protein digests were mixed and mapped together. In the mixing experiment, co-migrating peptides appear at full intensity, while peptides contributed by only one of the proteins appear diminished in intensity.

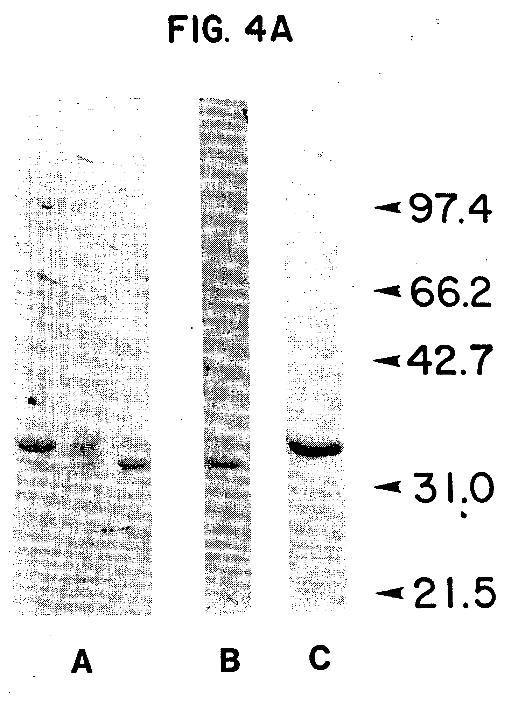

Figure 4A shows purification of pp35 and pp32. Panel A represents a Coomassie-stained gel of successive alternate fractions from the HPLC-anion exchange column. The figure shows the partial separation of pp35, pp32, and pp42 from one another. Panels B and C represent Coomassie-stained gels showing the homogeneous pp32 and pp35 obtained after HPLC hydroxylapatite chromotograpy. The numbered arrows indicate the positions of molecular weight standards in kDa.

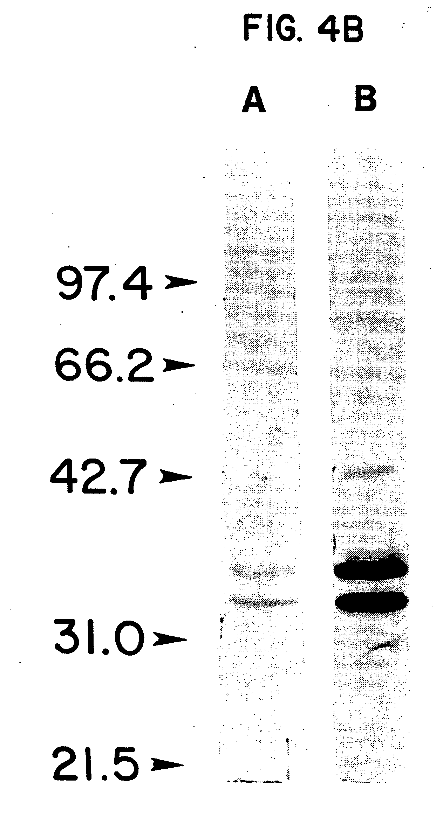

Figure 4B shows immunoreactivity of partially-purified pp35, pp32, and pp42. Panel B illustrates a Western blot of an HPLC anion exchange fraction similar to those shown in Figure 4A. Antibody to denatured pp35 identifies three species, pp42, pp35, and pp32. Panel A is a Coomassie-stained gel lane showing purified pp35 and pp32, and is included as a standard. The numbered arrows indicate the positions of molecular weight standards in kDa.

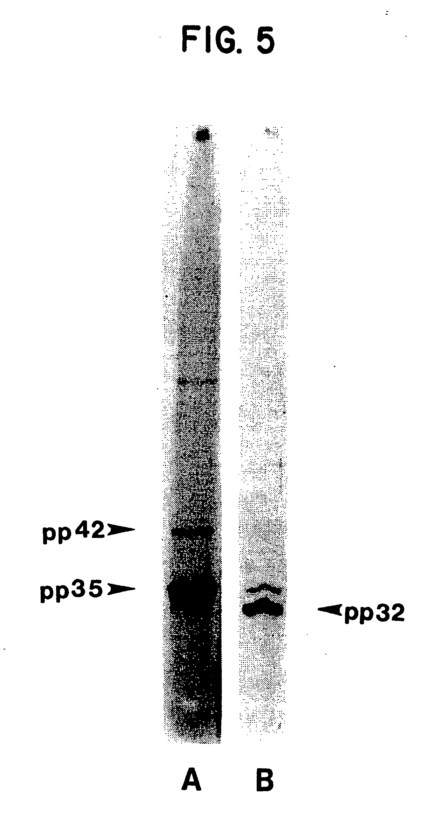

Figure 5 shows specificity of antibodies to native pp35 and pp32. The figure represents a Western blot of A₂₀ lysate using affinity-purified antibody to native pp35 (anti-pp35n) in lane A, and to native pp32 (anti-pp32n) in lane B. Anti-pp35n reacts primarily with pp35, but also slightly with pp32, pp42, and an unidentified band of approximately 68 kDa. Anti-pp32n reacts principally with pp32, but cross-reacts slightly with pp35.

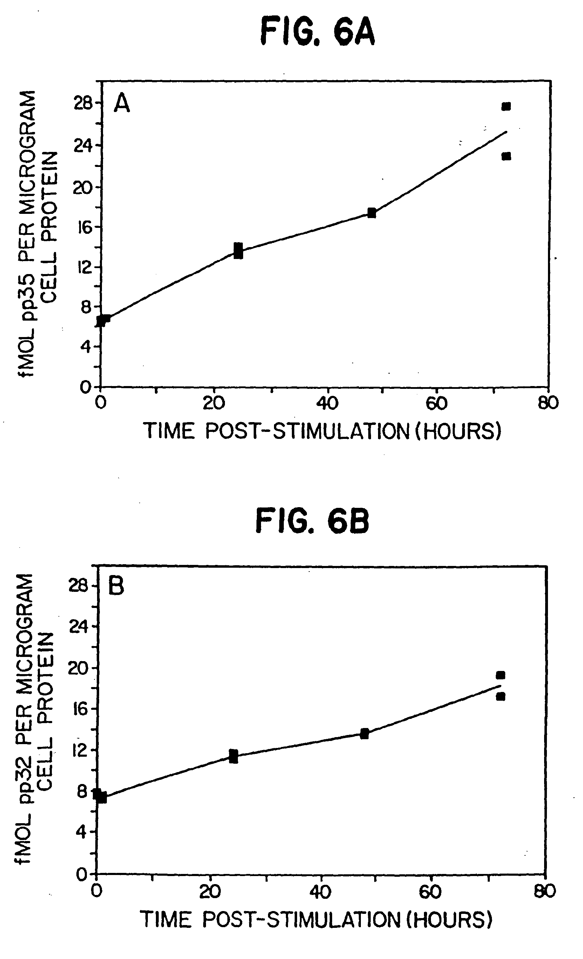

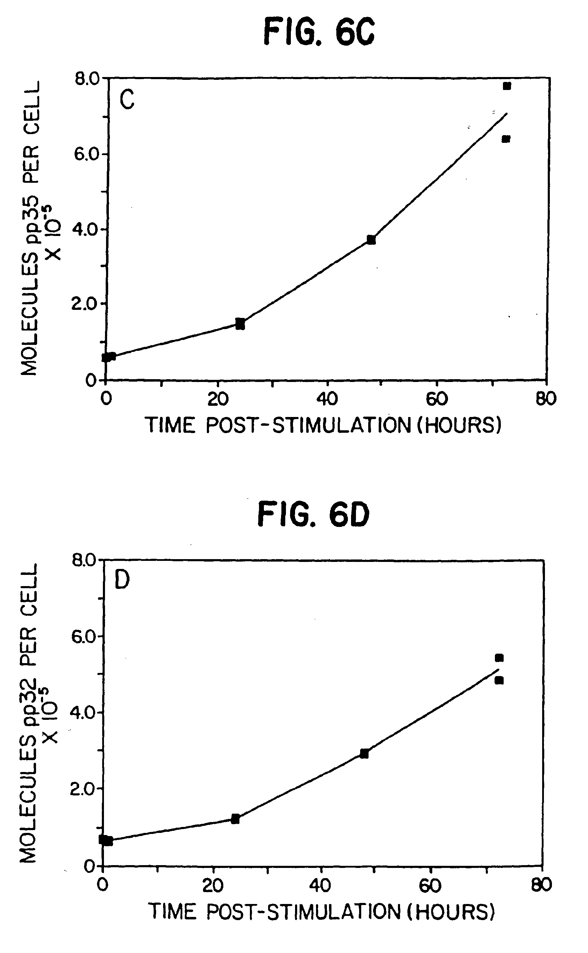

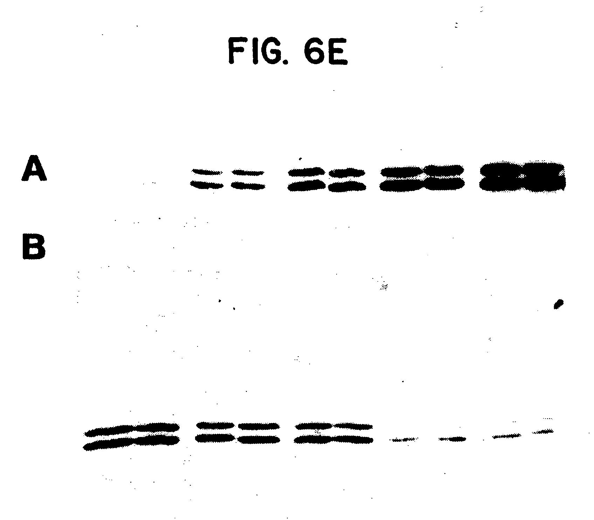

Figure 6 shows lipopolysaccharide stimulation of resting B cells. Purified small dense B cells were incubated with 40 µg/ml lipopolysaccharide from E. coli 0127:B-8 under conditions which consistently yield a 100-fold stimulation of thymidine incorporation measured by 72 h. Aliquots were removed at times 0, 1 h, 24 h, 48 h, and 72 h. The cell number and protein content of each aliquot was determined, and a portion of each was analyzed by immunoblotting with a cocktail of affinity-purified anti-pp35n and anti-pp32n and developing with ¹²⁵I-protein A. For quantitation, the experiment included a standard curve prepared with purified pp35 and pp32. The resultant autoradiographs were quantitated by computerized densitometric image analysis. Figure 6A shows the results for pp35 normalized to total cell protein (Panel A) and to cell number (Panel C), and for pp32 (Panels B and D). Figure 6B shows the autoradiographs from which the data in Figure 6A was obtained. Panel A represents the standard curve. Beginning at the left, the first pair of lanes illustrates the duplicate determinations for 31 ng each of pp35 and pp32. Each successive pair of lanes represents 62.5, 125, 250 and 500 ng. Panel B show the experimental autoradiographs. Beginning at the left, the first pair of lanes represents duplicate determinations for the 72 h time point. Each successive pair of lanes represents duplicate determinations for the 48 h, 24 h, 1 h, and 0 h time points.

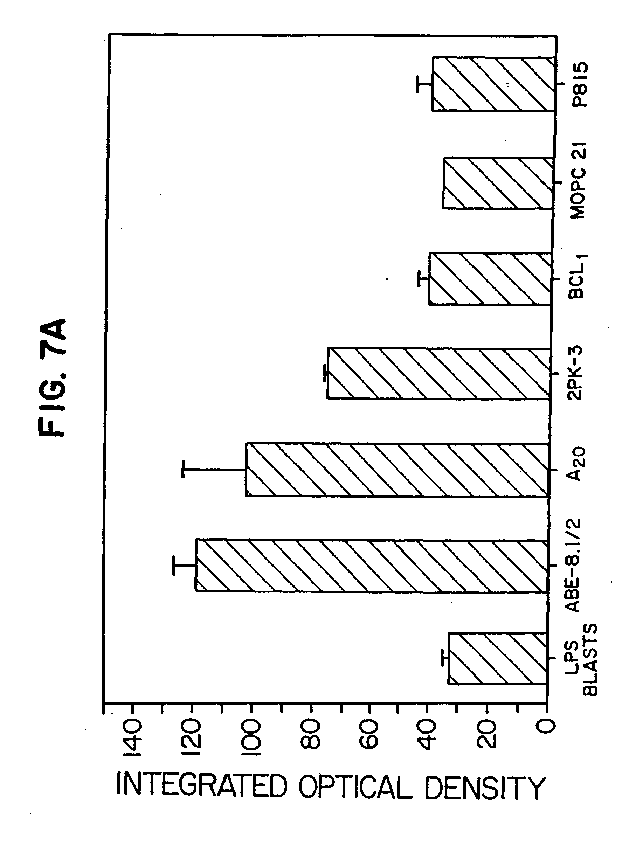

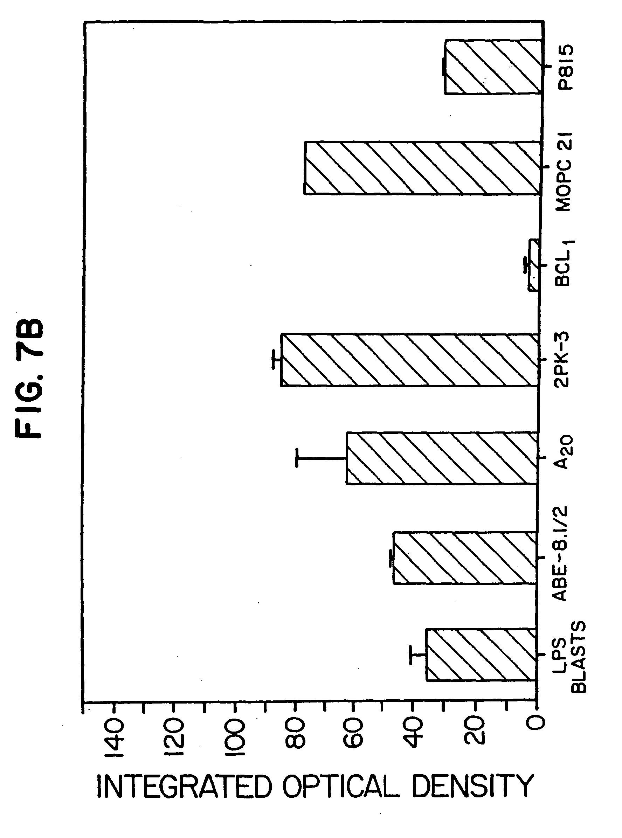

Figure 7 shows expression of pp35 and pp32 in cell lines. Cells from the indicated lines were processed for quantitative immunoblotting as described in the description of Figure 6. Equal amounts of cellular protein from each line were analyzed. The figures show the mean of duplicate determinations, and the error bars indicate the range; only a single determination was available for the MOPC 21 subclone (P3.6.2.8.1). Panels A and B show the results respectively for pp35 and pp32. ABE-8.1/2 is a pre-B cell line. A₂₀, 2PK-3, and BCL₁ are all B cell lines. MOPC 21 is a plasmacytoma.

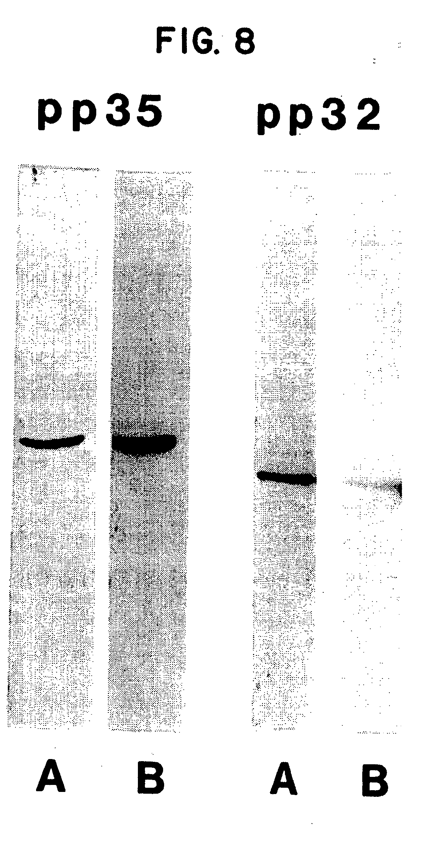

Figure 8 shows that pp35 and pp32 are phosphoproteins. A₂₀ cells were labeled for 4 h by incubation with ³²P orthophosphate in otherwise phosphate-free medium. pp35 and pp32 were isolated as described, electrophoresed on a 10% Laemmli gel, transferred to nitrocellulose, and analyzed by reactivity with antibodies to pp35 and pp32 (Lanes A) and by autoradiography (Lanes B). Antibody reactivity was detected colorimetrically.
Figure 9 shows pp35-related cDNA sequences. Three hundred bases of sequence from the open reading frame of cloned pp35 cDNA are shown together with the predicted amino acid sequence. The approximately 1 kb cDNA was subcloned into Bluescript and sequenced by the dideoxynucleotide method; approximately two-thirds of the insert has been sequenced to date. The underlined sequence exactly matches sequence obtained independently from a pp35 tryptic peptide isolated by reverse phase chromatography.

### DETAILED DESCRIPTION

It is a finding of the present invention that the expression of certain related mammalian nuclear proteins can be used to predict the malignant potential of lymphoid and epithelial tumors. Antibodies have been raised against the native forms of these proteins and they have been used as immunohistochemical reagents. The percentage of cells which stain positive with the reagents, the intensity of staining, and in some cases the location of the staining, correlates with the malignant potential of the lymphoid or epithelial tumors.

According to the present invention, there are three related proteins which are expressed by mammalian lymphoblastoid cells as well as human lymphoid and epithelial tumor cells. They are related immunologically and have also been shown to generate overlapping sets of cleavage polypeptides. The proteins have been found to be phosphoproteins and are thus termed pp42, pp35, and pp32, based on their molecular weights. While the phosphoproteins were originally found in mouse cells, homologs have been found in human cells. The human homologs are immunologically cross-reactive with antibodies which are raised against the murine proteins. Thus, such antibodies can be used in the practice of the present invention's diagnostic method; alternatively antibodies raised against the human homologs can be used. Antibodies raised against synthetic or cleavage polypeptides can also be used.

As mentioned above the mammalian proteins of the present invention are believed to be phosphorylated. It has been found that two of the proteins, pp32 and pp35 are substrates for the enzyme casein kinase II in vitro. Casein kinase II, or NII kinase (Rose et al., 1981, J. Biol. Chem., vol. 256, pp. 7468-7477), was initially described as a cyclic nucleotide-independent, heparin-sensitive kinase utilizing both ATP and GTP as phosphate donors; recent work suggests that there may be a family of casein kinase II-like enzymes (Kishimoto et al., 1987, J. Biol. Chem., vol. 262, pp. 1344-1351). Several convergent lines of evidence imply that casein kinase II plays a key role in the processes of cell proliferation and differentiation. Firstly, casein kinase II levels are elevated both in transformed cells (Brunati et al.. 1986, J. Immunol. vol. 127, pp. 2496-2501; Prowald et al., 1984, FEBS Letters, vol. 174, pp. 479-483) and during embryogenesis (Perez et al., 1987, Eur. J. Biochem., vol. 170, pp. 493-498; Schneider et al., 1986. Eur. J. Biochem., vol. 170, pp. 733-738); additionally, casein kinase II levels oscillate with the cell cycle (Carroll and Marshak, 1989, J. Biol. Chem., vol. 264, pp. 7345-7348) and undergo transient elevation during cell differentiation (Sommercorn and Krebs, 1987, J. Biol. Chem., vol. 262, pp. 3839-3843). Secondly, although casein kinase II substrates are not limited to the nucleus (Hathaway and Traugh, 1982, Curr. Top. Cell. Reg., vol. 21, pp. 101-127; Lees-Miller and Anderson, 1989, J. Biol. Chem., vol. 264, pp. 2431-2437; Wang et al., 1986, Biochem. Biophys. Acta, vol. 888, pp. 225-236; Grande et al., 1988. FEBS Letters, vol. 232, pp. 130-134), an ever-lengthening list of proteins which coordinate nuclear function are major substrates for casein kinase II (Matthews and Huebner, 1984, Mol. Cell. Biochem., vol. 59, pp. 81-99; Pfaff and Anderer, 1988, Biochem. Biophys. Acta, vol. 969, pp. 100-109) including RNA polymerases I and II (Duceman et al., 1981, J. Biol. Chem., vol. 256, pp. 10755-10758; Stetler and Rose, 1982, Biochemistry, vol. 21, pp. 3721-3728), DNA topoisomerases I and II (Durban et al., 1985, EMBO J., vol. 4, pp. 2921-2926; Ackerman et al., 1985, Proc. Natl. Acad. Sci., USA, vol. 82, pp. 3164-3168), high mobility group protein 14 (Walton et al., 1985, J. Biol. Chem., vol. 260, pp. 4745-4750) and C-proteins of heterogeneous nuclear ribonucleoprotein particles (Friedman et al., 1985, Biochem. Biophys. Acta, vol. 847, pp. 165-176: Holcomb and Friedman. 1984, J. Biol. Chem., vol. 259, pp. 31-40). Thirdly, growth factors such as insulin, epidermal growth factor, and insulin-like growth factor 1 stimulate casein kinase II activity in quiescent cells (Sommercorn et al. 1987, Proc. Natl. Acad. Sci. USA, vol. 84, pp. 8834-883; Klarlund and Czech, 1988, J. Biol. Chem., vol. 263, pp. 15872-15875; Ackerman and Osheroff, 1989, J. Biol. Chem., vol. 264, pp. 11958-11965). It appears that casein kinase II-mediated phosphorylation must in some way modulate those cellular functions forming the infrastructure of proliferation and differentiation. Whether pp42 is phosphorylated has not yet been determined. although pp42 is referred to herein as a phosphoprotein.

The 35 kD phosphoprotein of the present invention binds to myosin filaments. This property can be used as a means of purifying the phosphoprotein from other cellular proteins. The other proteins of the invention, of molecular weights 32 kD and 42 kD, do not bind to myosin, thus it appears that the myosin-binding domain is not present or accessible in these proteins. However, as discussed below, peptide mapping has indicated that other regions of the 35 kD structure are shared by the 32 and 42 kD proteins. This is confirmed by the fact that affinity purified antibodies which were raised against native pp35 are able to bind to both pp32 and murine pp42. No human homolog of murine pp42 has yet been observed.

According to the present invention molecular weight is an important identifying property of the phosphoproteins. The molecular weights are determined by SDS-polyacrylamide gel electrophoresis (SDS-PAGE). Such techniques are well known in the art. See Laemmli, 1970, Nature, vol. 227, pp. 680-685.

The amino acid sequences of proteolytic peptides pp35 were experimentally determined. These exactly correspond to the predicted amino acid sequence obtained by sequencing cDNA encoding a pp35-related protein. The predicted amino acid sequence is shown in Figure 9. This sequence can be used to synthesize polypeptides which can be used to raise antibodies as is known in the art. Alternatively synthetic polypeptides can be used (as can the whole protein) to compete with proteins present in a biological source for antibody binding. Such competition can be used to assure that antibody binding is specific and can also be used as a means of quantitating the amount of antigen present in a biological sample.

According to the present invention, the phosphoproteins can be isolated from any convenient mammalian source. One particularly convenient source is a mammalian lymphoblastoid cell line. A particularly preferred cell line is the murine cell line A₂₀. See Kim et al., 1979, J. Immunol., vol. 129, pp. 549-554. This cell line is available from the American Type Culture Collection in Rockville. Maryland. Phosphoproteins pp35 and pp32 can be collected from the nucleus of lymphoblastoid cell lines. Mammalian cell lines can be cultured according to methods known in the art using culture media and conditions which are well known.

Antibodies according to the present invention may be monoclonal or polyclonal. If polyclonal antibodies are employed, it is preferred that they be affinity purified to render them monospecific. As mentioned above, polyclonal antibodies which are raised against pp35 are also immunoreactive with pp32 and pp42. Of course since each of the proteins contains unique peptide sequences, it is likely that monoclonal antibodies could be raised which would not cross-react with the other members of the phosphoprotein family. That is to say that the peptide mapping data make it likely that there are unique as well as shared epitopes on each of the members of the phosphoprotein family.

Some antibodies according to the present invention are immunoreactive with the native forms of the phosphoproteins. Thus the proteins need not be denatured in order to render them suitable binding partners for the antibodies. However, the antibodies may be used to detect proteins in the denatured form, such as in Western blots and immunohistochemistry.

Other antibodies according to the invention are produced by immunizing experimental, antibody-producing animals with an immunogen which comprises a polypeptide. The polypeptide contains amino acid sequences corresponding to the phosphoproteins of the present invention, such as those shown in Figure 9. The immunogen may also contain other proteins such as keyhole limpet hemocyanin (KLH) which can be used to stimulate the animal to produce an immune response to a polypeptide which may be too small to do so on its own. Alternatively the immunogen may comprise all or a portion of another protein which is attached to the polypeptide and produced from a fusion gene. Such fusions may be used to express the polypeptide in bacterial or animal cells as is known in the art.

Methods have been developed to achieve preparations of apparent homogeneity of the phosphoproteins of the present invention. The methods involve lysis of the cells in a detergent and low-ionic strength buffer. Suitable detergents include Triton X-100 and other non-ionic detergents. The salt concentration of the buffers should preferrably be in the range of 5-50 mM. Purification of a crude cell lysate can be accomplished by sequential chromatography on DEAE-cellulose, HPLC anion-exchange, and HPLC hydroxylapatite columns. Fractions which are eluted from the chromatographs can be assessed by SDS-PAGE and by Western blotting using anti-pp35. for example. If desired an additional purification step can be employed in the purification of pp35 which involves the co-precipitation with cross-linked myosin filaments. Rabbit skeletal muscle is a suitable source of myosin. The phosphoproteins can be eluted from the myosin by extraction with 1 M KCl.

A substantially purified preparation of mammalian protein according to the present invention contains greater than about 75% of the desired phosphoprotein. Preferably the preparation contains greater than about 90% of the desired phosphoprotein. Most desireably the preparation contains at least about 95% of the desired phosphoprotein.

Also contemplated by the present invention as a diagnostic and prognostic tool are nucleic acid probes. Like the antibodies of the present invention, they can be used to quantitate the expression of gene products from the genes encoding pp32, pp35, and pp42. Gene products according to the invention include messenger RNA as well as protein or phosphoprotein. The probes are complementary to the mRNA sequences from which the three phosphoproteins are translated. Probes may also be used which vary slightly from the sequence of a particular phosphoprotein gene. For example, a probe may be derived from the murine phosphoprotein sequence and be useful for hybridization to human and other mammalian species genes. Alternatively, a probe may represent an allelic or polymorphic variant of a sequence. However, all probes must be sufficiently homologous to the phosphoprotein gene being probed to hybridize under the conditions of the well known techniques of Northern. Southern, and in situ hybridization.

Nucleic acid probes are generally labeled with a radioactive moiety. Alternatively they can be labeled with a fluorescent moiety or an enzyme which can cause a chromogenic substrate to change color. Nucleic acid probes are generally useful between about 15 and 1500 bases. Such probes may be synthesized using the sequence disclosed in Figure 9. Alternatively, sequences shown in Figure 9 can be used as a probe to detect other pp35 sequences or pp32 or pp42 sequences. These latter sequences can be used to derive probes for diagnostic uses. As the expression of pp35, pp32, and pp42 has been discovered to be elevated in relation to the degree of malignancy of a lymphoid or epithelial tumor, the steady state levels of the corresponding mRNA are almost certainly elevated also in such tumors. Particular methods for quantitating mRNA in tissue samples are known in the art, and any such method can be employed. It is possible that the elevated levels of the nuclear phosphoproteins of the invention are due to gene amplification. The probes of the present invention can be used in Southern hybridizations to detect such amplified genes.

Quantitative polymerase chain reaction (PCR) can be used to determine the steady state amount of mRNA in a tissue. See Wang et al. PNAS, vol. 86, pp. 9717-21, 1989. In order to practice this latter method to quantitate the amount of mRNA encoding the phosphoproteins of the invention, primers are used which are derived from the phosphoprotein gene sequences. As is known in the art, primers are complementary to opposite strands of a DNA duplex and flank a region of DNA to be amplified. To measure mRNA amounts cDNA is first made by reverse transcription and the cDNA is then amplified. PCR can also be used to quantitate genomic sequences which may be amplified in malignant tissues.

The diagnostic methods of the present invention are conveniently carried out using standard histological sections, such as paraffin-embedded sections. Either lymphoid or epithelial tissue can be used. Any of the preparations of antibodies which are reactive with pp32, pp35 and/or pp42 can be used to immunostain the histological sections. Immunostained sections can be analyzed to determine the percentage of cells which immunostain with the antibodies. As shown below in Table 1, the samples derived from increasingly more malignant tissues showed increasing percentages of immunostained cells. In the case of intermediate grade lesions, 60-70% of the cells are positive for the antibody stain. In the case of high grade lesions, greater than 90% of the cells are positive for the antibody stain.

Alternatively, in the case of staining with an anti-pp35 preparation, the localization of the immunostaining within the cells can be determined. Whereas in normal lymphoid tissue staining is restricted to germinal centers and small paracortical foci, in low grade lesions the staining is more intense and more widely distributed. In intermediate and some high grade lesions (diffuse large cell malignant lymphoma (diffuse histiocytic lymphoma)) the staining is in both the nucleus and the cytoplasm. In other high grade lesions (small non-cleaved cell malignant lymphoma (diffuse undifferentiated lymphoma)) the staining is solely in the nucleus. In the case of pp32 staining, normal tissues stain in the germinal centers and paracortex, whereas in the malignant tissues the staining is in the nucleus. Staining of pp32 does not appear in the cytoplasm in any of the lesions examined.

In yet another embodiment of the present invention, the immunostaining can be analyzed to determine the intensity of staining. Increasing intensity of staining correlates with increasing malignant potential.

The following examples are merely illustrative of the invention and do not limit the invention.

### Example 1

### pp35 co-sediments with crosslinked myosin.

pp35 co-sedimented with rabbit skeletal muscle myosin filaments. While this suggested a means of affinity purification, it required a convenient means to separate pp35 from myosin. Since amine crosslinkers uniformly inactivated the ability of myosin to bind pp35, myosin filaments were disulfide-crosslinked using o-phenanthroline and copper. Unlike native filaments, which disassemble in increasing salt concentrations, the crosslinked filaments permitted recovery of pp35 by elution in 1 M KCl. Figure 1, Lanes B and C show the respective pellet and supernatant resulting from incubation of crosslinked myosin in A₂₀ lysate, while lanes D and E respectively show the residual pellet and the material eluting from the myosin in 1 M KCl. The eluate contained a Coomassie-stainable 35 kDa band. pp35 was further purified by HPLC anion exchange chromatography, excised from a Laemmli gel (Laemmmli, 1970, Nature, vol. 227, pp. 680-685. and used to raise polyclonal antibodies. designated anti-pp35d (d designates that the immunogen was denatured).

Myosin Affinity Purification of pp35. Rabbit skeletal muscle myosin was purified as described (Margossian et al., 1982, Methods Enzymol., vol. 85, pp. 55-71) using DEAE cellulose chromatography in pyrophosphate buffers to remove the contaminating C-protein. For cross-linking, purified rabbit skeletal muscle myosin in 0.6 M KCl, 50 mM potassium phosphate, pH 6.5 at 15-20 mg/ml was diluted to 1 mg/ml in 10 mM sodium phosphate, pH 7.5 and incubated at 4° for 15 min. After incubation, the mixture was adjusted to 0.1 mg/ml myosin and brought to final concentrations of 1.7 mM (Cu(o-phenanthroline)₂, and 1% Triton X-100, pH 7.5. Generally, a lysate prepared from A₂₀ cells (8 mg total protein, see below) was incubated with 1 mg of cross-linked myosin for 30 min at 4°. The myosin was pelleted at 16,000 x g for 1 min, the supernatant removed, and the pellet washed three times in lysis buffer. The pellet was then resuspended in lysis buffer containing 1 M KCl and incubated at 4° for 15 min. After incubation, the myosin was again pelleted. The material eluted from cross-linked myosin was further purified by DEAE cellulose chromatography. The 1 M KCl extract was dialyzed into 20 mM KCl, 5 mM sodium phosphate, pH 7.6, with 0.1 mM henylmethylsulfonyl fluoride, 1 mM 2-mercaptoethanol, and 0.1% Triton X-100. The extract was loaded onto a 0.9 x 8 cm DEAE cellulose column equilibrated in the same buffer, which was then undercut with 0.3 M KCl and eluted with 0.5 M KCl in the same buffer. Following purification, the eluted material was re-dialyzed into the starting buffer and stored at 4°. A typical preparation yielded approximately 100 µg of total product, including impurities, from approximately 2.5 x 10⁹ cells grown in 11 of medium.

Antibody to denatured pp35 was raised in rabbits and affinity-purified. Initially, antigen was purified by electroelution from SDS-polyacrylamide gels essentially as described (Knowles and Bologna, 1983). To localize the band, 85 µg of pp35 kDa protein in 5 mM sodium phosphate, 20 mM KCl, 0.1% Triton X-100, pH 8.5, was reacted with a 100-fold molar excess of dansyl chloride. Derivatized protein was mixed with unreacted 35 kDa protein at a 1:9 ratio (w/w) and the electrophoretic bands visualized under ultraviolet light. Protein was eluted from the gel slices using a commercial apparatus (Isco Corp., Lincoln, Neb.). Initially, each of three female New Zealand white rabbits was injected at six subcutaneous sites with 50 µg of 35 kDa protein emulsified in phosphate-buffered saline containing 2% squalene. 50 µg trehalose dimycolate and 100 µg monophosphoryl lipid A (Ribi Immunochem Research, Inc., Hamilton, Montana). Each rabbit received a total volume of 1.8 ml. On day 19, the animals were boosted in similar fashion. On day 42, two of the rabbits were again boosted with 125 µg of protein directly excised from stained, neutralized gels and emulsified in the same adjuvant system. One rabbit responded, as determined by strong reactivity against the homologous antigen in a Western blot. This animal was bled on day 100. The resulting antiserum was purified on a column of pp35 kDa coupled to Reacti-Gel 6X (Pierce Chemical Co., Rockford, III.) with the same elution protocol as described above.

### Example 2

### Identification of pp42 and pp32.

Anti-pp35d was affinity purified as described above and used to examine A₂₀ lysates. In immunoblots, affinity-purified anti-pp35d reacted well with pp35, and, interestingly, with an additional 32 kDa protein designated pp32, as seen in Figure 2. During fractionation. this antibody also detected an additional low-abundance 42 kDa species, designated pp42 (Figure 4, v.i.).

Gel electrophoresis and Western blotting were performed as described. See Laemmli, 1970, Nature, vol. 227, pp. 680-685; Towbin et al., 1979, Proc. Natl. Acad. Sci. USA, vol. 76, pp. 4350-4354; Gershoni and Palade, 1983, Analyt. Biochem., vol. 131, pp. 1-15; Kuhajda et al., 1989, Proc. Natl. Acad. Sci. USA, vol. 86, pp. 1188-1192. In some instances, blots were visualized with a dye-based detection system. Immunoblots were transferred and processed through primary antibody incubation. Biotinylated goat anti-rabbit IgG (Vector Laboratories) was used as a secondary antibody, followed with intervening washes by an avidin-horse radish peroxidase conjugate (Vector Laboratories) and developed with a Biomeda Universal Substrate kit containing 3-amino, 9-ethyl carbazole (Biomeda Laboratories) used according to the manufacturer's directions.

Cell line A₂₀ was obtained from the American Type Culture Collection (ATCC), Rockville, Maryland. All media were supplemented with 2 mM L-glutamine, 100 u/ml penicillin, and 100 µg/ml streptomycin, and iron-supplemented newborn calf serum (Hyclone) at the indicated concentrations. All cells were grown in a humidified 5% CO₂ atmosphere at 37°. A₂₀ cells (Kim et al., 1979, J. Immunol., vo. 129, pp. 549-554) were grown in RPMI 1640 medium supplemented with 10% serum.

### Example 3

### Peptide mapping of pp42, pp35, and pp32.

The structural relationship between pp42, pp35, and pp32 was examined using high-resolution two-dimensional peptide mapping. Peptide mapping was performed using the Elder technique (Elder et al., 1977, J. Biol. Chem., vol. 252, pp. 6510-6515; Speicher et al., 1980, Proc. Natl. Acad. Sci. USA, vol. 77, pp. 5673-5677). Figure 3 shows the peptide maps of each species alone, and in combination with each of the other species. Note in the top row that the general pattern of the maps is similar, but that each map is unique. The combined maps show that while some peptides overlap, many are unique; in this system, the intensity of non-overlapping peptides is reduced relative to the individual maps, while overlapping peptides remain essentially unaltered. Importantly, one cannot simply derive the map of pp35 or pp32 from the map of pp42; likewise, the map of pp35 does not wholly contain the map of pp32. This suggests that the relationship between the proteins is more complex than one whereby the two smaller polypeptides would be derived from pp42 by proteolytic cleavage.

### Example 4

### Purification of pp35 and pp32.

The initial purification scheme using crosslinked myosin failed to precipitate pp32 and resulted in low yields. For these reasons, anti-pp35d was used to assay fractions during development of an alternative purification strategy, consisting of sequential detergent lysis in low-ionic strength buffer, DEAE-cellulose chromatography, HPLC anion-exchange chromatography, and HPLC hydroxylapatite chromatography. The Coomassie-stained gel shown in Figure 4a illustrates typical sequential fractions from the stage of HPLC anion exchange; this step fails to completely resolve pp35 and pp32 (panel A), and includes a faint 42 kilodalton band present in the middle and right-hand lanes. The Coomassie-stained lanes shown in panels B and C indicate that subsequent hydroxylapatite chromatography achieves essentially complete purity of greater than about 98%. Figure 4b, lane B shows that the faint 42 kDa band seen in Figure 4a is also immunoreactive with anti-pp35d. Generally, 100-200 µg of each protein could be purified from approximately 5 x 10⁹ cells, representing an estimated yield of around 20%.

In a typical preparation, 6 liters of A₂₀ cells were harvested by centrifugation at 600 x g, 4°, for 15 min then washed three times with unsupplemented RPMI 1640. For lysis, the cell pellet was resuspended with a pipette in 20 mM Tris-HCl, pH 7.5, 1 mM EDTA, 1% Triton X-100 (Pierce Chemical Co.), 10 mM sodium pyrophosphate, 2 mM sodium vanadate, 3 mM ATP, 50 mM NaF, 0.5 mM diisopropyl fluorophosphate (DFP), 0.1 mM phenylmethylsulfonyl fluoride (PMSF) to a density of 2 x 10⁸ cells/ml. The lysate was then centrifuged at 17,500 x g for 20 min at 4°, and the supernatant passed through a 0.2 µ filter before application to a 2.5 x 10 cm DEAE cellulose column (DE53, Whatman) pre-equilibrated in 150 mM NaCl, 20 mM Tris-HCl, pH 7.5 containing 2 mM sodium vanadate, 0.5 mM DFP, 0.1 mM PMSF. The column was then eluted with a 400 ml linear gradient to 600 mM NaCl in the same buffer with the eluate collected in 70 equal fractions. Fractions were electrophoresed on 10% Laemmli gels (Laemmli, 1970, Nature, vol. 227, pp. 680-685) which were further analyzed by Western blotting using anti-pp35d. Positive fractions containing pp42, pp35, and pp32 eluting around 400 mM NaCl were pooled and applied directly to an HR-5/5 MonoQ FPLC column (Pharmacia Fine Chemicals) pre-equilibrated in 150 mM NaCl, 20 mM Tris-HCL, pH 8.5, room temperature, 1 mM β-mercapto-ethanol, 0.1 mM PMSF at a flow rate of 2 ml/min. The column was then developed with a 60 ml gradient of 150 mM NaCl in the same buffer to 1.0 M NaCl in the same buffer at pH 7.0. Each 600 µl fraction was analyzed on Coomassie-stained Laemmli gels and by Western blotting as previously described. Under these conditions, pp42, pp35 and pp32 eluted at approximately 0.78 M NaCl. Following HPLC, fractions containing pp35 or pp32 were individually pooled and applied separately to a 30 x 4.6 mm MAPS HPHT analytical HPLC hydroxylapatite column (Bio-Rad) after 1:1 dilution in 10 mM sodium phosphate, pH 6.8 10 µM CaCl₂, 0.1 mM PMSF. The column was developed with an 18 ml linear gradient to 600 mM sodium phosphate, 10 µM CaCl₂, 0.1 mM PMSF at a flow rate of 0.3 ml/min. This purification protocal resulted in homogeneous preparations of pp35 and pp32 on Coomassie-stained Laemmli gels.

Purified pp35 and pp32 were used to raise another set of polyclonal antibodies. Using material purified as described above. 100 ug of either pp35 or pp32 in 2 ml of complete Freunds adjuvant were injected into four subcutaneous sites in each of two Pasteurella-free New Zealand White rabbits on Day 0. On Day 14, the rabbits were boosted with the same amount of antigen in incomplete Freund's adjuvant, with bimonthly bleedings commencing on Day 28. Antibody production was monitored by Western blotting. Initially, an IgG fraction was prepared by loading serum aliquots onto a 5 ml protein A-Sepharose CL4B (Pharmacia) column eluted with 0.1 M glycine pH 2.7 and collected intc 0.5 M sodium phosphate buffer pH 7.5. Fractions were concentrated up to 25 mg/ml IgG by vacuum dialysis against phosphate buffered saline containing 0.1 mM PMSF, 1 mM β-mercaptoethanol. These antibodies, designated anti-pp35n and anti-pp32n, were affinity-purified as described below, and characterized for use in quantitative immunoblotting, immunoprecipitation, and immunohistochemical localization studies.

For affinity-purification of anti-pp35n, an affinity column was prepared by concentrating the protein on an 0.4 ml hydroylapatite column (Fast-Flow, Calbiochem) to 600 µg/ml in the eluting 0.4 M sodium phosphate buffer, then reacting 600 µg of pp35 with 1 ml of EAH Sepharose CL4B (Pharmacia) and 2 mg each of 1-ethyl-3-(3-dimethylaminopropyl) carbodiimide HCl and N-hydroxysulfosuccinimide (Pierce Chemical Co.) for 6 h at room temperature under constant stirring. Any remaining reactive groups were capped by incubation with 0.5 M Tris HCl, pH 7.5, for 2 h. After washing, the derivatized resin was stored in phosphate-buffered saline containing 12 mM sodium azide. A similar column was prepared using 200 µg of pp32 coupled to 1.4 ml of resin. To affinity-purify the antibodies, 0.8 ml of IgG solution were run into a column and incubated for 1 h at room temperature. The column was washed with 15 ml of 2x phosphate-buffered saline containing 0.05% Tween 20, and the antibody eluted with 3 ml of 0.1 M glycine pH 2.7. The antibody was collected into 1 ml of 0.5M sodium phosphate, pH 7.5 and concentrated by vacuum dialysis. Figure 5 illustrates the specificity of these antibodies in Western blots of A₂₀ cell lysates; lane A shows that anti-pp35n reacts primarily with pp35, while lane B shows that anti-pp32n reacts primarily with pp32. The cross-reactivities are informative as well. Anti-pp35n shows minor cross-reaction with pp32, pp42, and an unidentified band, while anti-pp32 cross-reacts with pp35. This pattern demonstrates that pp35 and pp32 are unique proteins sharing some epitopes in common with each other, and, in the case of pp35, with pp42. Moreover, these immunochemical data independently support the conclusions drawn from the analysis of peptide maps.

### Example 5

### Induction of pp35 and pp32 in resting B cells.

The difference between the staining observed in normal tissues and in A₂₀ cells reflects differences in proliferative and functional state of the cell samples. Purified small, dense, resting B cells express relatively low levels of pp35 and pp32 until they are activated and driven into proliferation by a well-characterized polyclonal B cell stimulator, bacterial lipopolysaccharide (See Rabin et al., 1986, J. Exp. Med., vol. 164, pp. 517-531; Coffman et al.. 1986, J. Immunol., vol. 136, pp. 4538-4541). The data, shown in Figure 6a, demonstrate that over the course of 72 hours, the expression of pp35 rose 12-fold from 5.9 x 10⁴ to 7.1 x 10⁵ copies per cell (panel C), while pp32 increased 7.4-fold from 7.0 x 10⁴ to 5.2 x 10⁵ per cell (panel D). Because cell stimulation involves a global increase in cell size, these data were also normalized to total cellular protein so as to reflect specific induction over and above the general increase. Shown in Panels A and B, these data demonstrate a 3.8-fold specific induction of pp35, and a 2.3-fold induction of pp32. Preliminary studies measuring tritiated thymidine incorporation by resting B cell cultures as a function of time and LPS concentration (data not shown) yielded stimulation patterns over 72 h consistent with those reported in the literature. Expression of pp35 and pp32 was quantified by ¹²⁵I-protein A immunoblotting in conjunction with computerized image analysis, which yielded an integrated optical density for each band on an autoradiograph; integrated optical density values were converted to protein measurements through a parallel calibration curved of purified pp35 and pp32. Figure 6b illustrates the immunoblots from which Figure 6a was derived.

Six to eight week old BALB/C mice were obtained from Charles River. Small dense B cells were isolated from spleen on Percoll gradients essentially as described (Rabin et al., 1986, J. Exp. Med., vol. 164, pp. 517-531; Coffman et al., 1986, J. Immunol., vol. 136, pp. 4538-4541). T cells were removed using a cocktail of hybridoma culture supernatants including C3PO (Vidovic et al., 1984. J. Immunol., vol. 132, pp. 1113-1117), an anti-Ly-1, JIJ (Bruce et al., 1981, J. Immunol., vol. 127, pp. 2496-2501), an anti-Thy 1.2, RL172 (Ahmed et al., 1988, J. Viral., vol. 62, pp. 2102-2106), an anti-CD4, and 3155 (Sarmiento et al., 1980, J. Immunol., vol. 125, pp. 2665-2672), an anti-CD8, together with 10% Low-Tox-M rabbit complement (Cedarlane Laboratories). The T cell reagents were the gracious gift of Dr. Drew Pardoll. To measure activation by lipopolysaccharide, cells at 1.5 x 10⁵ cells per well were plated into 96 well tissue culture plates in a total volume of 220 µl per well. Cells were activated by incubation at 37° for 48 h with increasing doses of lipopolysaccharide (LPS W E.coli 0127:B-8, Difco laboratories). The cells were then pulsed with 1 µCi/well of ³H thymidine (ICN) for 16 h, then harvested with a cell-harvester onto Whatman glass-fiber filters presoaked in 10 mM thymidine. This resulted in a dose-dependent stimulation of thymidine incorporation of up to 140-fold over a range 0.08 - 50 µg/ml LPS. Cell viability was determined by amido schwarz dye exclusion, and was 95% for stimulated B cells at 72 h, and 50% for unstimulated cells.

To determine pp35 and pp32 content by quantitative Western blotting, resting B-cells were incubated at the above concentration in T-150 culture flasks for 0 h, 1 h, 24 h, 48 h, and 72 h in the presence of 40 µg/ml LPS. At each time point, cells were counted harvested, lysed by resuspension in 150 µl of 20 mM Tris-HCl, pH 7.5, 1 mM EDTA, 1% Triton X-100, 0.1 mM PMSF, 0.5 mM DFP. The supernatants were then collected by centrifugation at 16,000 x g for 10 min at 4° and stored at -80°; separate aliquots were reserved for determination of total protein by BCA protein assay (Pierce Chemical Co.). Similar duplicate amounts of total cellular protein for each time point were separated on 10% Laemmli gels along with a standard curve prepared from a mixture of purified pp35 and pp32 calibrated by BCA protein assay. The standard curve consisted of 31 ng, 62.5 ng, 125 ng, 250 ng, and 500 ng each of pp35 and pp32. The gels were transferred to nitrocellulose and the blots probed in the same solution of a mixture of affinity purified anti-pp35n and anti-pp32n at 5 µg/ml in Tris-saline containing 3% bovine serum albumin for 2 h followed. after intervening washes, by ¹²⁵I protein A at 3 x 10⁶ cpm/ml in albumin-Tris saline for 2 h. The blots were washed together extensively in Tris-saline, and exposed on the same piece of Kodak XAR-5 film in a cassette containing a Cronex Lightning Plus intensifying screen (Du Pont). The bands seen on the autoradiogram were quantitated by computed densitometry using a Loats image analysis system. The integrated optical density values obtained for the standard values of pp35 and pp32 were plotted against the amount of protein.

### Example 6

### pp35 and pp32 expression in lymphoid cell lines.

In populations of neoplastic cell lines, pp35 and pp32 are expressed at high levels (Figure 7). In the majority of cell lines, these levels exceed those seen in normal B cells stimulated with LPS for 72 h. To a first approximation, there is a reciprocal relationship between expression of pp35 and pp32 in that less differentiated lines tend to express higher levels of pp35 than pp32, while for more differentiated lines the reverse is true. There are at least two possible explanations for the discordant case of BCL₁: pp32 expression may be low; or pp32 itself may be immunologically altered. While these data are open to a number of interpretations, one explanation is that pp35 and pp32 assume different functions in the cell nucleus which are linked to different states of proliferation or differentiation.

2PK-3, a BALB/C B cell lymphoma (Lanier et al., 1981, J. Immunol., vol. 127, pp. 1691-1697), and ABE-8.1/2, a BALB/C pre-B cell lymphoma (Burchiel and Warner, 1980, J. Immunol., vol. 124, pp. 1016-1021), were grown in Dulbecco's modified Eagle's medium (DMEM) supplemented with 4.5 g/l glucose, 50 µM 2-mercaptoethanol, and 10% serum. P815, a DBA/2 mastocytoma (Ralph et al., 1976, J. Exp. Med., vol. 143, pp. 1528-1533), and P3.6.2.8.1, a subline of the BALB/C plasmacytoma MOPC 21 (Knopf et al., 1973, Eur. J. Immunol., vol. 3, pp. 251-259), were grown in DMEM with 10% serum. BCL₁ Clone CW13.30-383, a BALB/C B cell leukemia (Brooks et al., 1984, J. Immunol., vol. 133, pp. 3133-3137). was grown in RPMI 1640 with 10% serum.

### Example 7

### pp35 and pp32 are phosphoproteins.

The initial evidence that pp35 and pp32 are phosphoproteins came from simultaneous autoradiography and immunoblotting of these proteins after purification from cells labeled with ³²p as orthophosphate. In one experiment, A₂₀ cells were labeled for 4 h in otherwise phosphate-free medium prior to purification. For each of the pp35 and pp32 panels of Figure 8, lane A illustrates a colorimetric immunoblot developed with either anti-pp35n or anti-pp32n, and lane B represents an autoradiograph of the blot. Both pp35 and pp32 are phosphoproteins in vivo. In this experiment, complete coincidence of the immunoreactivity of each protein with the radioactive band was observed. Moreover, ³²P-labeled pp35 and pp32 are radiochemically pure, so that measurements of radiochemical activity truly reflect incorporation into each protein.

### Example 8

### Casein kinase II phophorylates pp35 and pp32.

The potential role of various kinases in pp35 and pp32 phosphorylation was studied in vitro by their ability to phosphorylate native and dephosphorylated pp35 or pp32. Labeled pp35 contains phosphoserine and phosphothreonine, while labeled pp32 contains only phosphoserine; alkaline phosphatase quantitatively dephosphorylates ³²P-phosphoserine in both pp35 and pp32.

A₂₀ cells were labeled in vivo. Approximately 5 x 10⁹ A20 cells were washed once and resuspended at 2 x 10⁷ cells/ml in phosphate-free minimal essential medium (Gibco) supplemented with 10% iron-supplemented calf serum previously dialyzed against medium, 2 mM L-glutamine, 100 U/ml penicillin, 100 µg/ml streptomycin, and 1x non-essential amino acids solution. The cells were incubated in humidified 5% CO₂ atmosphere at 37° with 15 mCi of carrier-free ³²P as orthophosphate (Amersham) for 3 h. Following incubation, pp35 and pp32 were purified as described above.

Studies of several kinases in vitro showed that both pp35 and pp32 are substrates for casein kinase II. The results strongly suggest a physiologic role for casein kinase II in pp35 phosphorylation, and for a related kinase in pp32 phosphorylation. Casein kinase II consistently yielded the highest degree of ³²P incorporation into dephosphorylated pp35 and pp32, rephosphorylating pp35 to a level of 0.76 mol/mol (Table I).

**Table I**

| | | |
|---|---|---|
| Casein Kinase II | pp35, dephosphorylated | 0.76 mol/mol ± 0.03 |
| | pp35, native | 0.05 mol/mol ± 0.01 |
| | pp32, dephosphorylated | 0.08 mol/mol ± 0.01 |
| | pp32, native | <0.01 mol/mol |
| | | |
| Protein Kinase A | pp35, dephosphorylated | <0.02 mol/mol |
| | pp35, native | <0.02 mol/mol |
| | pp32, dephosphorylated | <0.02 mol/mol |
| | pp32, native | <0.02 mol/mol |
| | | |
| Protein Kinase A | pp35, dephosphorylated | <0.01 mol/mol |
| | pp35, native | <0.01 mol/mol |
| | pp32, dephosphorylated | <0.01 mol/mol |
| | pp32, native | <0.01 mol/mol |
| | | |
| EGF Receptor Kinase | pp35, dephosphylated | no incorporation |
| | pp32, dephosphorylated | <0.01 mol/mol |

In contrast, the catalytic subunit of protein kinase A, protein kinase C, and EGF receptor kinase all failed to promote significant levels of incorporation, and showed little sensitivity to whether the substrate proteins were dephosphorylated or not. As would be predicted for casein kinase II activity, phosphorylation of both pp35 and pp32 by casein kinase II utilized GTP as a substrate and was completely inhibited by heparin.

Rephosphorylation of the dephosphorylated phosphate turnover sites was investigated with protein kinase C from rat-brain, the catalytic subunit of cAMP-dependent protein kinase (Sigma), casein kinase II from bovine thymus, and A431 cell epidermal growth factor receptor kinase, generously provided as a solution of 30 µg/ml of EGF receptor in buffer containing 50 µM EGF by Dr. Wolfgang Weber (Institut fur physiologische Chemie, Universitat Hamburg).

Casein kinase II was purified as described (Zandomeni et al.. 1988, FEBS Letters, vol. 235, pp. 247-251). Aliquots of fractions were screened for casein kinase II activity in 30 µl final volumes of 20 mM Tris HCl at pH 7.5 160 mM NaCl, 8 mM MgCl₂, 0.1 mM DTT, 0.1 mM GTP with gamma-labeled ³²P GTP (Amersham) at a final specific activity of 1 µCi per nanomole. 1.0 mg/ml partially hydrolyzed and dephosphorylated casein (Sigma), in the presence and absence of the casein kinase II inhibitor 5,6-dichloro-1-beta-D-ribofuranosylbenzimidazole (DRB, Sigma) at a concentration of 100 µM, added from a stock solution in 50% dimethyl sulfoxide. After incubation at 30° for 10 min, reactions were terminated by the addition of 500 µl of ice-cold 25% TCA. Fractions showing kinase activity 50% inhibitible by DRB were selected for pooling. The final enzyme preparation showed the expected subunit structure on Coomassie-stained Laemmli gels, utilized GTP, showed partial inhibition by DRB, and showed complete inhibition by 1 µg/ml heparin.

Dephosphorylation of pp35 and pp32 was carried out by incubation with 1 unit of alkaline phosphatase from bovine intestinal mucosa (Sigma, Type VII) per µg of protein in 20 mM Tris HCl at pH 8.0, 1 mM MgCl₂, 0.1 mM ZnCl₂ for 1 h at room temperature. The reaction mixture was loaded onto a 0.2 ml hydroxylapatite column (high resolution, Calbiochem), previously equilibrated in 20 mM sodium phosphate pH 7.5. The column was eluted sequentially with 0.4 ml each of 100 mM, 200 mM and 450 mM sodium phosphate, pH 7.5. Alkaline phosphatase eluted at 100 mM sodium phosphate, while pp35 or pp32 eluted at 450 mM. Proteins labeled in vivo with 32p as orthophosphate were used to estimate the extent of dephosphorylation. While the ³²P as orthophosphate were used to estimate the extent of dephosphorylation. While the ³²P label was quantitatively removed from pp32, pp35 retained approximately one-third of the original label in the form of phosphothreonine.

Protein kinase C was purified using elements of two protocols (Walton et al., 1987, Analyt. Biochem., vol. 161, pp. 425-437; Woodgett and Hunter, 1987, J. Biol. Chem., vol. 262, pp. 4836-4843). Briefly, eleven rat brains were harvested, immediately frozen in liquid nitrogen, and homogenized with a Brinkmann Polytron in 150 ml of 10 mM Tris HCl at pH 7.5, 10 mM EGTA, 5 mM EDTA, 0.1% (v/v) β-mercaptoethanol, 4 µg/ml leupeptin, 0.5 mM diisopropylfluorophosphate, 0.1 mM phenylmethyl sulfonyl fluoride, and 6 µg/ml soybean trypsin inhibitor. The homogenate was centrifuged for 30 min at 10,000 x g. The supernatant was filtered through three layers of cheesecloth into DE53 (Whatman) pre-equilibrated in 20 mM Tris HCl at pH 7.5, 1 mM EDTA, 0.1% (v/v) β-mercaptoethanol and batch adsorbed for 30 min while stirring at 4°. The resin was poured into a 2 x 20 cm column which was then eluted with a 11 gradient of 20 mM NaCl to 300 mM NaCl in the same buffer. The column fractions were screened for phorbol ester stimulated activity by incubating 5 µl aliquots with 5 µl of 1 mg/ml histone H-1 (Boehringer-Mannheim) in a final volume of 20 µl containing 20 mM HEPES, pH 7.5, 10 mM MgCl₂, 0.5 mM CaCl₂, 50 µg/ml freshly sonicated phosphatidylserine, 0.1 mM ATP with gamma-labeled ³²P-ATP (Amersham) at a final specific activity of 1 µCi per nanomole, and, in some samples, phorbol myristic acetate at 5 µg/ml. After initiation by the addition of substrate stock, the reactions were stopped after 5 min at 30° by the addition of 500 µl of 20% ice cold 20% trichloroacetic acid (TCA). Precipitates were incubated at 4° for 20 min, then centrifuged for 5 min at 16,000 x g. The pellets were washed twice with 20% TCA and then counted by Cerenkov counting. Positive fractions were selected on the basis of PMA-stimulated phosphorylation of histone H-1, pooled, and then brought to 1.5 M NaCl by the addition of solid NaCl. This pool was then loaded onto a 10 ml phenyl Sepharose column (Pharmacia) pre-equilibrated in 20 mM Tris HCl at pH 7.5, 2 mM EDTA, 2 mM EGTA, 1 mM dithiothreitol, 1.5 M NaCl. The column was eluted with a 200 ml linear gradient of 1.5 M NaCl to O M NaCl in the same buffer. Positive fractions were pooled and applied to a 10 ml protamine-agarose column (Pharmacia) equilibrated in 20 mM tris Hcl at pH 7.5, 2 mM EGTA, 2 mM EDTA 1 mM DTT 0.1 mM PMSF, 0.5 mM DFP 0.1 M NaCl pH 7.5. The column was eluted with a 160 ml linear gradient of increasing salt from 0.5 M to 1.5 M NaCl. Purified protein kinase C activity comigrated with an 80 kDa band on Coomassie-stained Laemmli gels; PMA increased histone H-1 phosphorylation by 15 to 18 fold over baseline samples without PMA in typical preparations. The final yield was approximately 40 µg. The enzyme was stored stably for several months at -80° in buffer containing 16% glycerol and 0.02% Triton X-100.

Protein kinase C assays were carried out for 30 min at 37° with 0.5 µg of the 80 kDa protein kinase C isoenzyme in 20 mM HEPES at pH 7.5, 10 mM MgCl₂, 0.5 mM CaCl₂, 50 µg/ml freshly sonicated phosphatidylserine, and 0.1 mM ATP containing gamma-labeled ³²P ATP (Amersham) at a final specific activity of 5 µCi per nanomole, in the presence and absence of 5 µg/ml PMA. Generally, 1 to 5 µg of pp35 or pp32 were tested. Reaction products were analyzed on Laemmli gels and on two dimensional gels (O'Farrell, 1979, J. Biol. Chem., vol. 250, pp. 4007-4021) gels using histone H1 (Boehringer) as a positive control substrate.

Protein kinase A assays utilized the catalytic subunit of CAMP-dependent protein kinase from bovine heart (Sigma). Reactions were carried out by incubating 50 units of enzyme with 5 µg of either pp35 or pp32 for varying times at 30° in 120 µl volumes of 50 mM HEPES at pH 7.4, 25 mM NaCl. 10 mM MgCl₂, 1 mM EDTA, 0.1 mM β-mercaptoethanol, and 0.1 mM ATP containing gamma-labeled ³²P ATP at a final specific activity of 1 µCi per nanomole. 1 µg histone H1 served as a positive control substrate. Reaction products were analyzed as above.

Casein-Kinase II assays were carried out by incubating 1 µg enzyme with 1 to 5 µg pp35 or pp32 for varying times at 30° in 20 mM Tris Hcl at pH 7.9, 8 mM MgCl₂, 0.1 mM DTT and 0.1 mM GTP containing gamma-labeled ³²P GTP at a final specific activity of 1 µCi per nanomole. Partially dephosphorylated and hydrolyzed casein from bovine milk (Sigma) served as positive control substrate. 1 µg/ml of heparin in the reaction mixture completely inhibited phosphate incorporation into casein, pp35, and pp32. Reaction products were analyzed as above.

The epidermal growth factor receptor assays were carried out by incubating 0.15 µg kinase with 1 to 5 µg pp35 or pp32 for varying times at 30° in 100 µl of 20 mM HEPES at pH 7.5, 1 mM MnCl₂, 5 mM MgCl₂, 50 mM EGF, and 0.1 mM ATP containing gamma-labeled ATP at a final specific activity of 1 µCi per nanomole. The autophosphorylation of the receptor served as a positive control for the kinase activity. Reaction products were analyzed as above.

The amount of phosphate transferred from ATP or GTP to pp35 or pp32 was calculated from Cerenkov counts of the corresponding excised bands from Coomassie-stained gels. Raw Cerenkov counts were converted to moles of phosphate using an experimental specific activity value in Cerenkov counts per minute per nMol phosphate obtained from triplicate counts of 5 µl aliquots of reaction mixtures of known nucleotide concentration; care was taken to closely approximate the sample geometry used to measure the activity of the gel slices. These measurements were combined with previous triplicate measurements of protein substrate concentration by the BCA assay (Pierce Chemical Co.) to calculate the stoichiometry of phosphorylation at the susceptible sites. The background counts subtracted from each measurement were obtained by counting gel slices of comparable area from each lane from areas with no protein. Kinase reactions were carried out in triplicate for varying time points up to 2 h to assure completion. Casein kinase II reactions were complete by 15 min, while protein kinase A, protein kinase C, and the EGF receptor kinase all showed small, gradual increases over the two hour period with no obvious plateau. Two hours were therefore arbitrarily chosen as a cutoff for the determination of phosphorylation stoichiometry for these latter kinases.

### Example 9

### HeLa cells contain pp35 and pp32.

HeLa cells, a human cervical epithelioid carcinoma cell line, (available from the ATCC) appear to contain pp35 and pp32. An immunoblot of a total HeLa cell hypotonic detergent lysate was prepared essentially as described for A₂₀ cells above. Antibodies to denatured, gel-purified pp35 which recognize both pp35 and pp32 react with a diffuse band at approximately 33 kDa; in Lighter exposures the heavy band resolves into two closely-spaced components. The experiment clearly indicates the presence of cross-reactive species of the expected molecular weights in a human cell line, but does not clearly establish the number of species or degree of relationship with murine lymphoid pp35 or pp32.

### Example 10

### pp35 and pp32 immunostaining correlates with increased malignant potential.

Human tissues react with antibodies to native murine pp35 and p32, showing increased staining frequency, increased staining intensity, and altered subcellular distribution with increasing malignancy.

Paraffin-embedded sections of human lymphoid tissue were stained with affinity-purified antibodies to native pp35 and pp32. Staining was evaluated semi-quantitatively by two independent observers. In a diffuse large cell lymphoma, the neoplastic cells show prominent nuclear staining while the normal lymphocytes are negative. Adenomatous colonic epithelium shows prominent nuclear anti-pp35 staining in virtually every cell, in contrast to the limited staining of crypts seen in normal colon. In contrast, invasive adenocarcinoma of the colon shows predominantly cytoplasmic pp35 staining, while pp32 staining remains nuclear. This change from nuclear to cytoplasmic distribution is highly reminiscent of the association of c-abl transforming activity with a relocation from nucleus to cytoplasm (Van Etten, et al., Cell, vol. 58, pp. 669-678, 1989).

Table 2 illustrates the results of a screen of human lymphomas with anti-pp35 and anti-pp32, which demonstrates that increased staining frequency and intensity and altered distribution are associated with increased malignancy. Lymphomas were examined for pp35 and pp32 staining independent of diagnosis, then ranked in increasing order of frequency of staining. The result was a ranking which predicted the level of virulence suggested by the diagnosis.

**TABLE 2**

| pp35 and pp32 Staining in Normal and Neoplastic Lymphoid Tissue | | | | | | |
|---|---|---|---|---|---|---|
| | pp35 | | | pp32 | | |
| LESIONS | % Positive | Intensity | Location | % Positive | Intensity | Location |
| LOW GRADE LESIONS | | | | | | |
| Small Lymphocytic | 40-50% | +1 | Nucleus | | NOT DONE | |
| | | | | | | |
| Malignant Lymphoma (Well-Differentiated Lymphocytic) | staining in tumor only. Weak, infrequent staining of normal small Lymphocytes | | | | | |
| | | | | | | |
| Follicular Lymphoma Small Cleaved Predominant (Poorly Differentiated Lymphocytic Lymphoma) | 40-50% | +/- | Nucleus | 40-50% | +1 | Nucleus |
| | | | | | | |

| INTERMEDIATE GRADE LESION | | | | | | |
|---|---|---|---|---|---|---|
| | | | | | | |
| Follicular Lymphoma Large Cell Predominant (Nodular Histiocytic Lymphoma) | 60-70% | 2+ | Cytoplasm | 60-70% | +3 | Nucleus |
| | | 1+ | Nucleus | | | |
| | Staining described is for large cell component. Small cells aboved +/- nuclear staining in 40-50% of cells. | | | Small cells showed 40-50% +1 nuclear staining. | | |

| HIGH GRADE LESIONS | | | | | | |
|---|---|---|---|---|---|---|
| | | | | | | |
| Diffuse-Large Cell | >90% | 3+ | Nucleus | >90% | +3 | Nucleus |
| Malignant Lymphoma (Diffuse Histiocytic Lymphoma) | Focal | 2+ | Cytoplasm | | | |
| | Small, normal Lymphocytes negative Small, normal Lymphocytes negative | | | | | |
| Small Noncleaved Cell Malignant | >90% | 3+ | Nucleus | >90 | 3+ | Nucleus |
| Lymphoma (Diffuse Undifferentiated Lymphoma) | Small, normal lymphocytes negative Small, normal lymphocytes negative | | | | | |

| NORMAL TISSUES | | | | | | |
|---|---|---|---|---|---|---|
| Tonsil % Reactive | 50-60% | 1+ | Nucleus | 50-60% | 1+ | Nucleus |
| Lymph Node (Identical Findings) | | | Germinal | | | Germinal |
| | | | Centers | | | Centers |
| | | | | | | |
| | 30-60% | 1+ | Nucleus Paracortex | 30-40% | 1+ | Nucleus Paracortex |
| | | | | | | |
| | Occasional small faci of 3+ with 70% positive nuclear staining in paracartax. | | | Occasional small foci of 3+ with 70% positive nuclear staining in paracortex. | | |

### Example 11

### pp35 cDNA is cloned and partially sequenced.

Screening an oligo dT-primed A₂₀ **λ** gt11 cDNA library with a 42 base oligonucleotide guessmer (Sambrook, et al., Molecular Cloning: A Laboratory Manual, Cold Spring Harbor Laboratory Press, 1989, pp. 11.1,1-11.16) derived from pp35 peptide sequence recovered a pp35-related cDNA predicting additional independent pp35 peptide sequence. The major pp35 phosphopeptide sequence L-L-P-Q-L-S-Y-L-D-G-Y-D-D-E containing a casein kinase II phosphorylation site was backtranslated to the best guess 42 base oligonucleotide sequence using previously described codon preference rules (Lathe, J. Mol. Biol., vol. 183, pp. 1-12, 1985). The resultant oligonucleotide, 5'-CTGCTGCCCCAGCTGTCCTACCTGGATGGCTATGATGATGAG, hybridizes with a 1.3 kb RNA species in Northern blots of A₂₀ cell RNA.

Approximately 600,000 **λ** gt11 plaques were screened with end-labeled oligonucleotide and the filters washed at moderate stringency, yielding 23 cDNA clones which remained positive through tertiary plaque purification. The first pp35 cDNA to be subcloned into Bluescript™ and partially sequenced by double-stranded dideoxynucleotide sequencing is approximately 1kb and contains predicted protein sequences identical to an independently sequenced pp35 peptide. A portion of the sequence of the pp35 related cDNA is shown in Figure 9.

The cDNA and predicted peptide sequences were compared to all nucleotide and protein sequences in the Genbank and EMBL libraries using the FASTA program of the University of Wisconsin in GCG sequence analysis package, and using the TFASTA program to compare predicted amino acid sequence to translations of the nucleotide sequences. No close matches were found, confirming that pp35 is distinct from previously described nuclear proteins. pp35 cDNA does contain interesting homologies of 30 to 50% over 60 to 100 nucleotide stretches with such molecules as human calcyclin, human c-myc germ line protooncogene, human calmodulin, and human U2 snRNP, however the true extent and significance of these homologies is at present unknown. No homology has been found in the determined sequence to erythrocyte protein 4.1, even though some preparations of anti-4.1 antibodies cross-react with denatured pp35. Curiously, there is no such cross-reactivity with native pp35. This case is reminiscent of synapsin I, which also cross-reacts with anti-protein 4.1 antibodies but is unrelated at the sequence level (Baines and Bennett, (1985), Nature, vol. 315, pp. 410-413; McCaffery and DeGennaro (1986), EMBO J., vol. 5, pp. 3167-3173; and Conboy, et al., (1986), Proc. Natl. Acad. Sci. USA, vol. 83, pp. 9512-9516.)

## Claims

1. A preparation of antibodies which specifically binds to a mammalian protein comprising an amino acid sequence as shown in Figure 9.

2. An antibody which specifically binds a human nuclear protein in native conformation, said human protein being purified from human cells by lysing the cells in hypotonic medium containing non-ionic detergent and salt concentration from 5-50 mM, recovering a lysate, and fractionating the lysate by multi-stage anion exchange chromatography, said human protein having a molecular weight of about 32 kDa as measured by SDS-PAGE under denaturing conditions, said human protein being detected in Western blot by affinity-purified polyclonal antibodies which specifically bind a murine protein in native conformation, said murine protein containing the amino acid sequence of Figure 9.

3. The antibody according to claim 1 or 2, wherein the antibody is in an isolated polyconal antiserum, a preparation of purified polyclonal antibodies, or a preparation containing one or more monoclonal antibodies.

4. The antibody according to any of the preceding claims, wherein the antibody does not specifically bind to human calcyclin polypeptides, human c-myc germ line protooncogene polypeptides, human calmodulin polypeptides, or human U2 snRNP polypeptides.

5. A diagnostic method for predicting malignant potential of lymphoid and epithelial tumors, comprising:
obtaining a sample from human lymphoid or epithelial tissue; and
detecting, in the sample, an antigen which specifically binds an antibody of any one of the preceding claims.

6. A diagnostic method for predicting malignant potential of lymphoid and epithelial tumors, comprising:
obtaining a sample from human lymphoid or epithelial tissue, and
contacting the sample with antibodies which specifically bind a mammalian protein found in the nucleus of A₂₀ cells having a molecular weight of about 35 kDa as determined by SDS-PAGE, wherein said mammalian protein is a substrate for casein kinase II *in vitro.*

7. A diagnostic method for predicting malignant potential of lymphoid and epithelial tumors, comprising:
obtaining a sample from human lymphoid or epithelial tissue;
immunostaining the sample with antibodies which specifically bind a mammalian protein found in the nucleus of A₂₀ cells having a molecular weight of about 35 kDa as determined by SDS-PAGE; and analysing the immunostained sample by
(i) quantitating the percentage of cells which immunostain with the antibodies, increasing percentages of immunostained cells correlating with increasing malignant potential;
(ii) scoring the intensity of immunostaining, increasing intensity of immunostaining correlating with increasing malignant potential; or
(iii) scoring for the percentage of cytoplasmic immunostaining, increasing cytoplasmic immunostaining correlating with increasing malignant potential.

8. The method according to claim 5, wherein the tissue is a lymphoma.

9. The method according to claim 5, wherein the tissue is a carcinoma.

10. The method according to claim 9, wherein the tissue is a colon carcinoma.
